# EUROPÄISCHE PATENTSCHRIFT

(11) **EP 1 696 989 B1**
(45) Veröffentlichungstag und Bekanntmachung des Hinweises auf die Patenterteilung: **09.01.2013**
(21) Anmeldenummer: 04818394.1
(22) Anmeldetag: 11.11.2004
(51) Int. Cl.: A61M 16/06, A62B 18/08

(54) **KOPFBANDEINRICHTUNG FÜR EINE ATEMMASKE, SOWIE VERFAHREN ZUR HERSTELLUNG DERSELBEN**
HEADBAND DEVICE FOR AN OXYGEN MASK, AND METHOD FOR THE PRODUCTION THEREOF
DISPOSITIF BANDEAU SERRE-TETE POUR UN MASQUE RESPIRATOIRE ET PROCEDE POUR PRODUIRE UN TEL DISPOSITIF

(30) Priorität: 11.11.2003 DE 10352608; 11.11.2003 DE 10352607
(43) Veröffentlichungstag der Anmeldung: 06.09.2006
(60) Teilanmeldung: 11170495.3
(73) Patentinhaber: MAP Medizin-Technologie GmbH, 82152 Martinsried (DE)
(72) Erfinder: LANG, Bernd, 82166 Gräfelfing (DE); BIENER, Achim, 85445 Aufkirchen (DE); BECHTEL, Martin, 21423 Winsen/Luhe (DE); VÖGELE, Harald, 82131 Gauting (DE); STAUFFENBERG, Caspar, Graf, 82131 Gauting (DE)
(74) Vertreter: Vossius & Partner
(86) Internationale Anmeldenummer: PCT/EP2004/012811
(87) Internationale Veröffentlichungsnummer: WO 2005/046776

(56) Entgegenhaltungen:
- EP-A- 1 356 843
- WO-A-02/07806
- WO-A1-02/47749
- US-B1- 6 470 886

## Beschreibung

Die Erfindung betrifft eine Kopfbandeinrichtung zur Applikation einer Atemmaske im Gesichtsbereich eines Anwenders. Weiterhin betrifft die Erfindung auch ein Verfahren zur Herstellung einer derartigen Kopfbandeinrichtung. Es wird weiterhin auch eine ein Kopfband umfassende Applikationseinrichtung für eine Atemmaske beschrieben.

Atemmasken finden insbesondere im medizinischen bzw. therapeutischen Bereich zur Behandlung schlafbezogener Atmungsstörungen Anwendung. Durch derartige Atemmasken ist es möglich, einem Anwender ein atembares Gas, insbesondere gefilterte Umgebungsluft, auf einem Druckpegel zuzuführen, der über dem Umgebungsdruck liegt. Durch die derart bei erhöhtem Druck vorgenommene Atemgasversorgung wird es möglich, im Bereich der oberen Atemwege eine pneumatische Schienung zu erreichen und hierdurch etwaigen Obstruktionen in diesem Atemwegsbereich vorzubeugen. Die derart zur Behandlung schlafbezogener Atmungsstörungen verwendeten Atemmasken werden vom Anwender über die gesamte Ruhe- bzw. Schlafphase getragen. Diese Atemmasken werden üblicherweise über eine Kopfbandanordnung am Kopf des Anwenders fixiert. Diese Kopfbandanordnungen können einen oberen Kopfbandabschnitt sowie einen unteren Kopfbandabschnitt umfassen, wobei über den oberen Kopfbandabschnitt entsprechende Haltekräfte auf eine Stirnauflageeinrichtung der Atemmaske aufgebracht werden können. Durch den unteren Kopfbandabschnitt kann die Atemmaske gegen den Umgebungsbereich der Nase, den Nasenrückenbereich sowie den Oberlippenbereich gedrängt werden.

Die WO-A-02/47749 betrifft eine Kopfbandeinrichtung zum Sichern und Positionieren einer zur Behandlung einer Atmungsstörung geeigneten Maske, die aus einem Verbundmaterial aufweisend Polyurethanschaum aufgebaut ist. Die Kopfbandeinrichtung umfasst einen Hinterabschnitt mit unteren und oberen Bändern die zum Hinterabschnitt verbunden sind. Die Bänder haben relativ schmale Bandenden, wobei unteren Bänder bezüglich des Hinterabschnitts nach untern versetzt sind. Ein Schnelllösemechanismus nahe der Vorderseite des Gesichts befestigt die Kopfbandeinrichtung mit der Maske.

Der Erfindung liegt die Aufgabe zugrunde, eine Kopfbandeinrichtung zu schaffen, die unter fertigungstechnischen Gesichtspunkten in vorteilhafter Weise herstellbar ist und die sich durch einen hohen Trage- und Handhabungskomfort auszeichnet.

Diese Aufgabe wird gemäß einem ersten erfindungsgemäßen Lösungsansatz gelöst durch eine Kopfbandeinrichtung nach Anspruch 1.

Die Schließmittel umfassen eine Schlitteneinrichtung, die an der Kopfbandeinrichtung in unterschiedlichen Schließstellungen arretierbar ist. Die Schlitteneinrichtung kann hierbei einen Arretiermechanismus umfassen, zur Arretierung der Schlitteneinrichtung an der Arretierstruktur.

Dadurch wird es auf vorteilhafte Weise möglich, eine sich durch Polstereigenschaften auszeichnende Kopfbandeinrichtung ohne erhebliche Zuschnittsverluste als Serienteil zu fertigen.

Gemäß einer besonders bevorzugten Ausführungsform der Erfindung wird das zur Bildung des Bandkorpus vorgesehene Kunststoffmaterial durch Einspritzen des Kunststoffmaterials in einen entsprechenden Formraum zu dem Bandkorpus geformt Der Bandkorpus ist in vorteilhafter Weise derart ausgebildet, dass dieser im Bereich seiner Außenflächen verhautet ist. Der Verhautungseffekt kann erreicht werden, indem der entsprechende Forminnenraum mit einer Beschichtung versehen wird. Die Beschichtung kann insbesondere durch einen Pulverbeschichtungsvorgang oder durch Einlegefolien erreicht werden.

Gemäß einer besonders bevorzugten Ausführungsform der Erfindung ist der Bandkorpus mit einer zugsteifen Einlage versehen. Diese zugsteife Einlage ist vorzugsweise aus einem thermoplastischen Kunststoffmaterial, insbesondere einem Nylon- oder Polyamidmaterial, gefertigt. Die in den Bandkorpus, eingebundene Einlage kann zumindest abschnittsweise derart ausgebildet sein, dass diese abschnittsweise eine formstabile Einlage bildet. Die Polstereigenschaften, insbesondere die Polsterdicke des Bandkorpus, kann derart variieren, dass insbesondere in Bandzonen mit erhöhter Flächenpressung dickere Polsterstärken vorherrschen.

Gemäß einer besonders bevorzugten Ausführungsform der Erfindung ist der Bandkorpus mit Schließmitteln versehen zur Ankoppelung eines Bandlaschenabschnitts oder Maskenbefestigungselementen in einstellbarer Weise. Dadurch wird es möglich, über die Kopfbandeinrichtung den Maskenanpressdruck präzise einzustellen. Die Schließmittel umfassen vorzugsweise eine Arretierstruktur, die integral mit der Kopfbandeinrichtung ausgebildet ist. Die Arretierstruktur kann derart ausgebildet sein, dass ein Arretiereffekt auf Grundlage einer Kraft und/oder formschlüssigen Koppelung mit einer entsprechenden Gegenstruktur erreicht werden kann. Es ist möglich, die Arretierstruktur so auszubilden, dass diese insbesondere integral mit der zugkraftaufnehmenden bzw. zugsteifen Einlage ausgebildet ist. Die zugsteife Einlage kann derart ausgebildet sein, dass diese ein bestimmtes Biegeverhalten aufweist. Dieses bestimmte Biegeverhalten kann insbesondere durch die Ausbildung von Aussparungen in der zugsteifen Einlage sowie durch Abstimmung der Dicke der zugsteifen Einlage erreicht werden.

Die eingangs angegebene Aufgabe wird gemäß einem weiteren Aspekt der vorliegenden Erfindung auch gelöst durch ein Verfahren zur Herstellung einer Kopfbandeinrichtung, bei welchem im Rahmen eines ersten Kunststoffspritzschrittes zur Bildung einer zugsteifen Einlage ein entsprechendes Kunststoffmaterial in einen zur Bildung der Einlage konfigurierten Formwerkzeugraum eingespritzt wird und im Rahmen eines nachfolgenden Kunststoffspritzschrittes ein porenbildendes Kunststoffmaterial in einen erweiterten Formraum derart eingespritzt wird, dass das porenbildende Material unter Bildung eines Polsterabschnitts die zugsteife Einlage zumindest abschnittsweise ummantelt

Weiterhin wird auch eine Applikationsvorrichtung für eine Atemmaske beschrieben, insbesondere für Atemmasken zur Behandlung schlafbezogener Atmungsstörungen.

Die Applikationsvorrichtung ermöglicht eine zuverlässige Fixierung einer Atemmaske und zeichnet sich durch einen hohen Anwendungskomfort aus.

Die Applikationsvorrichtung für eine Atemmaske beishaltet eine Kopfbandeinrichtung, die sich in Applikationsposition um den Hinterkopfbereich eines Anwenders erstreckt, wobei die Kopfbandeinrichtung mit einer Stützstruktur ausgestattet ist und die Stützstruktur aus einem Material gefertigt ist, das wenigstens einmal temporär in einen Zustand bringbar ist, in welchem die Kopfbandanordnung zumindest abschnittsweise an die Hinterkopftektur des Anwenders individuell anpassbar ist.

Dadurch wird es auf vorteilhafte Weise möglich, eine Kopfbandanordnung zu schaffen, durch welche ein, auf die individuelle Kopfform des Maskenanwenders vorteilhaft abgestimmter Verlauf der sich zur Atemmaske hin erstreckenden Kopfbandabschnitte gewährleistet ist. Weiterhin wird es auch in besonders vorteilhafter Weise möglich, die über die Kopfbandabschnitte aufgebrachten Zugkräfte unter einer geringen Flächenpressung in den Hinterkopfbereich des Maskenanwenders einzuleiten.

Vorzugsweise ist die Kopfbandanordnung derart gestaltet, dass die Stützstruktur auf Ohrhöhe, oder im Bereich des Halswirbelauslaufs auf dem Hinterkopfbereich des Anwenders aufsitzt.

Die Stützstruktur ist bevorzugt aus einem, nach Erwärmung auf eine Temperatur von vorzugsweise wenigstens 30°C plastisch verformbaren Material gefertigt. Die Erwärmung der Stützstruktur kann beispielsweise in einem Wasserbad oder durch Heißluft erfolgen.

Die Stützstruktur ist vorzugsweise aus einem Kunststoffmaterial, insbesondere einem thermo-plastischen Kunststoffmaterial, gefertigt. Die Stützstruktur kann so ausgebildet sein, dass diese eine Versteifungslage bildet, die beispielsweise in noch nicht vollständig ausgehärtetem Zustand an die Hinterkopfwölbung des Anwenders anpassbar ist. Die Stützstruktur kann auf ihrer, in Applikationsposition einem Anwender, zugewandten Seite, mit einer Polsterung versehen sein. Diese Polsterung kann aus einem Schaumstoff- und/oder Vliesmaterial gebildet sein.

Insbesondere bei einer Ausgestaltung der Stützstruktur als gewölbtes Plattenelement ist diese vorzugsweise mit Durchbrechungen versehen. Hierdurch wird die Dampfdurchlässigkeit der Stützstruktur erhöht und einer Überfeuchtung der Kopfbandeinrichtung vorgebeugt. Es ist möglich, die Stützstruktur derart auszubilden, dass diese lösbar mit der Kopfbandeinrichtung gekoppelt ist. Dadurch wird es auf vorteilhafte Weise möglich, die Kopfbandeinrichtung von der Stützstruktur zu trennen und die Kopfbandeinrichtung separat zu reinigen.

Die Stützstruktur ist bevorzugt derart ausgebildet, dass diese Armabschnitte aufweist, die sich von einem Hauptauflageflächenabschnitt ausgehend entlang der Kopfbandabschnitte erstrecken.

Die Stützstruktur kann derart ausgebildet sein, dass diese einen unteren Randabschnitt mit zwei unteren Schenkeln aufweist, die sich in Applikationsposition zum Wangenknochen bzw. in einen unter dem jeweiligen Ohrläppchen liegenden Bereich hin erstrecken, zur Führung von unteren Bandabschnitten der Kopfbandanordnung. Dadurch wird es auf vorteilhafte Weise möglich, den Verlauf der unteren Kopfbandabschnitte über den Wangenbereich des Anwenders vorteilhaft auf die individuelle Kopfform abzustimmen.

Die Sützstruktur kann vorzugsweise derart ausgebildet sein, dass diese einen oberen Randabschnitt mit zwei oberen Schenkeln aufweist, die sich von einer, im Nackenbereich liegenden Ausgangszone ausgehend, in einer über den Ohrenbereich weisenden Ausrichtung erstrecken.

Weitere Einzelheiten und Merkmale der Erfindung ergeben sich aus der nachfolgenden Beschreibung in Verbindung mit der Zeichnung. Es zeigt:
- **Figur 1a**: eine Seitenansicht eines Anwenders mit einer durch die erfindungsgemäße Kopfbandeinrichtung applizierten Atemmaske,
- **Figur 1b**: eine Skizze zur Erläuterung einer erfindungsgemäßen Kopfbandeinrichtung,
- **Figur 2a**: eine Querschnittansicht eines Bandkorpus ohne zugsteife Einlage,
- **Figur 3**: eine Bandkorpus-Querschnittskizze zur Erläuterung einer Variante mit zugsteifer Einlage,
- **Figur 4**: eine Skizze zur Erläuterung eines an einem erfindungsgemäßen Kopfband ausgebildeten Arretierabschnitts,
- **Figur 5a**: eine Schnittansicht zur Erläuterung des Querschnittprofils eines Bandkorpus mit integral mit diesem ausgebildeter Arretiereinrichtung,
- **Figur 5b**: eine, der strukturgemäß Figur 5a, zugeordnete Schlitteneinrichtung,
- **Figur 6a**: eine weitere Querschnittsvariante eines Bandkorpus mit darin ausgebildeter zugsteifer Einlage sowie integral mit der zugsteifen Einlage ausgebildeter Arretiereinrichtung,
- **Figur 6b**: eine Skizze zur Erläuterung eines der Arretiereinrichtung nach Figur 6a zugeordneten Schlitteneinrichtung,
- **Figur 7**: eine perspektivische Skizze zur Erläuterung weiterer Einzelheiten eines aus einem Schaummaterial gefertigten Kopfbands für eine Atemmaske mit einer zugsteifen Einlage und einer integral mit dieser ausgebildeten Arretiereinrichtung,
- **Figur 8**: eine Skizze zur weiteren Funktionsbeschreibung der Kopfbandeinrichtung gemäß Figur 7.
- **Figur 9**: eine Seitenansicht eines Atemmaskenanwenders mit einer, an diesem über eine Kopfbandanordnung applizierten Atemmaske,
- **Figur 10**: eine Ansicht des Hinterkopfbereichs eines Maskenanwenders zur Erläuterung einer bevorzugten Gestaltung der Kopfbandanordnung in diesem Bereich,
- **Figur 11**: eine Skizze zur Erläuterung des Aufbaus einer erfindungsgemäßen, von einer Atemmaskenanordnung getrennten, Applikationsvorrichtung,
- **Figur 12**: eine Skizze zur Erläuterung einer bevorzugten Querschnittsgestaltung der Applikationsvorrichtung im Bereich der Stützstruktur.

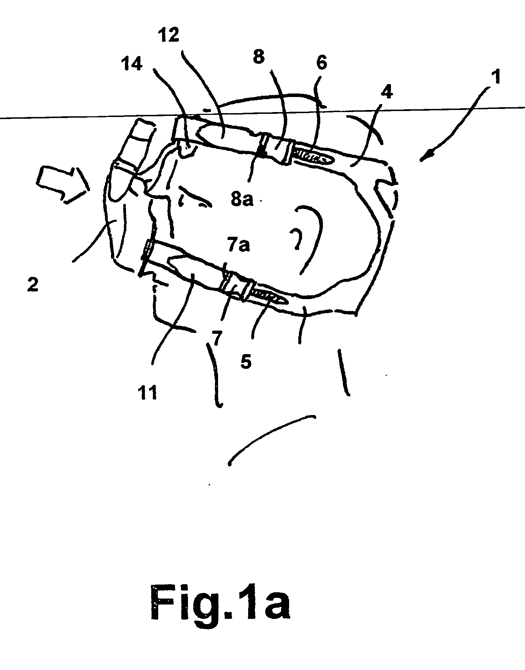

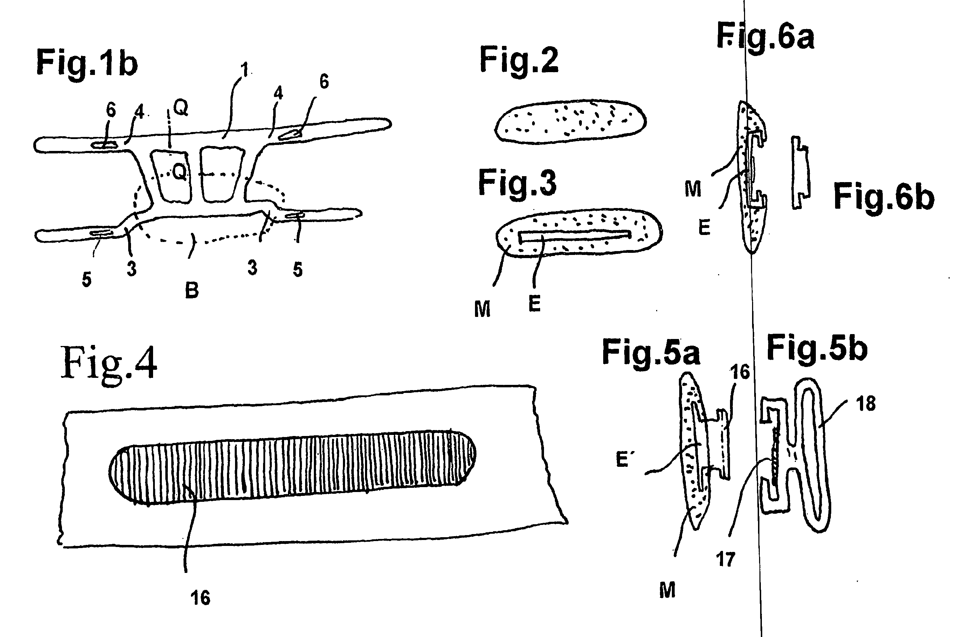

Der in Figur 1 dargestellte Atemmaskenanwender trägt eine, durch eine erfindungsgemäße Kopfbandeinrichtung 1 nasal applizierte Atemmaske 2. Die Fixierung der Atemmaske 2 erfolgt über untere Kopfbandabschnitte 3 und obere Kopfbandabschnitte 4. Die Kopfbandeinrichtung 1 ist aus einem geschäumten Kunststoffmaterial gefertigt, indem dieses im Rahmen eines Kunststoffspritzschrittes in einen entsprechenden Formraumabschnitt eingespritzt wurde.

Die Kopfbandeinrichtung 1 ist mit Arretiereinrichtungen 5, 6 versehen, durch welche Fixiermittel 7, 8 in einstellbar veränderbarer Weise an dem jeweiligen Kopfbandabschnitt 3, 4 festlegbar sind. Die Fixiermittel 7, 8 weisen jeweils Laschenabschnitte 7a, 8a auf, wobei durch diese Laschenabschnitte 7a, 8a weitere, zur Atemmaske 2 vordringende Bandabschnitte 11, 12 hindurchgeführt sind.

Die Kopfbandeinrichtung 1 ist derart ausgebildet, dass der untere Kopfbandabschnitt 3 sich aus dem Nackenbereich, unter den Ohren über den Wangenbereich hin zur Atemmaske erstreckt. Der obere Kopfbandabschnitt 4 erstreckt sich aus dem oberen Hinterkopfbereich in den Stirnbereich des Maskenanwenders. Durch den unteren Kopfbandabschnitt 3 und den oberen Kopfbandabschnitt 4 werden die zur Applikation der Atemmaske 2 erforderlichen Haltekräfte aufgebracht.

Durch den unteren Kopfbandabschnitt 3 wird imwesentlichen der Anpressdruck der Atemmaske 2 auf den Umgebungsbereich der Nase des Maskenanwenders bestimmt. Durch die im oberen Kopfbandabschnitt 4 herrschende Zugkraft wird im Wesentlichen der Anpressdruck einer Stimauflageeinrichtung 14 auf die Stirn des Maskenanwenders bestimmt.

In Figur 1b ist skizzenhaft eine, in eine Ebene ausgebreitete Kopfbandeinrichtung 1 gezeigt. Diese Kopfbandeinrichtung umfasst die oberen Kopfbandabschnitte 4 sowie die vorangehend genannten unteren Kopfbandabschnitte 3. Die Kopfbandeinrichtung 1 ist aus einem geschäumten Material gefertigt und mit einer zugsteifen Einlage versehen. Die zugsteife Einlage ist in dem hier durch Punktlinien gekennzeichneten Bereich B derart ausgebildet, dass diese um eine zur Auflagefläche imwesentlichen senkrechte Achse eine besonders hohe Torsionssteifigkeit aufweist. Hierdurch wird es möglich, im Bereich der unteren Kopfbandabschnitte 3 eine Bandkröpfung zu realisieren, durch welche die unteren Kopfbandabschnitte 3 in vorteilhafter Weise unter dem Ohrbereich des Anwenders auf dessen Wangenbereich hingeführt werden können. Die Kopfbandeinrichtung 1 ist, wie angedeutet, durch integral mit dieser ausgebildeten Arretierstrukturen 5, 6 versehen, auf die nachfolgend noch näher eingegangen werden wird.

Figur 2b zeigt eine Ausführungsform der erfindungsgemäßen Kopfbandeinrichtung, die insgesamt aus einem Poren bildenden Kunststoffmaterial gefertigt ist.

Figur 3 zeigt eine Querschnittsskizze Q/Q zur Erläuterung einer Variante des Bandkorpus mit darin vorgesehener zugsteifer Einlage E. Die zugsteife Einlage ist aus einem thermo-plastischen Kunststoffmaterial, beispielsweise Nylon oder Polyamid, gefertigt. Die zugsteife Einlage E ist in ein Schaummaterial M eingebettet. Das Schaummaterial M ist an die zugsteife Einlage E angespritzt.

Figur 4 zeigt eine Skizze zur Erläuterung einer, gemäß einer besonders bevorzugten Ausführungsform der Erfindung, integral mit der zugsteifen Einlage E ausgebildeten Arretierstruktur 16. Die Arretierstruktur 16 ist als querverzahnter Bahnabschnitt ausgebildet und kann insbesondere den in Figur 5a dargestellten Querschnitt aufweisen. Der Bahnkörper der Arretierstruktur 16 ist derart ausgebildet, dass dieser eine Führungseinrichtung für ein Schlittenelement bildet. Die Rastposition des Schlittenelements an der Bahneinrichtung ist in einstellbarer Weise veränderbar.

Figur 5b zeigt skizzenhaft eine mögliche Variante eines Schlittenelements, wie dies auf die Bahneinrichtung gemäß Figur 5a bzw. gemäß Figur 4 aufsetzbar ist. Das Schlittenelement umfasst einen hier nicht näher dargestellten Arretiermechanismus 17, der mit der Arretierstruktur 16 selektiv in Eingriff bringbar ist. An dem Schlittenelement ist ein Laschenabschnitt 18 ausgebildet zur Aufnahme eines Bandabschnitts 11, 12 wie in Figur 1a dargestellt.

Figur 6a zeigt eine weitere Querschnittsvariante der erfindungsgemäßen Kopfbandeinrichtung im Bereich der Arretiereinrichtung. Der Kopfbandkorpus umfasst auch hier eine, aus einem geschäumten Material gefertigte, Polsterlage M sowie eine darin eingebettete zugsteife Einlage E. Die zugsteife Einlage bildet auch hier Teil einer Arretiereinrichtung, die abweichend von der Variante nach Figur 5a weitgehend versenkt im Bandkorpus aufgenommen ist. In die, in den Bandkorpus hineinversenkte Bahneinrichtung ist ein Schlittenelement einsetzbar, das über einen, hier ebenfalls nicht näher dargestellten Arretiermechanismus, in einstellbar veränderbarer Weise an der entsprechende Führung festlegbar ist.

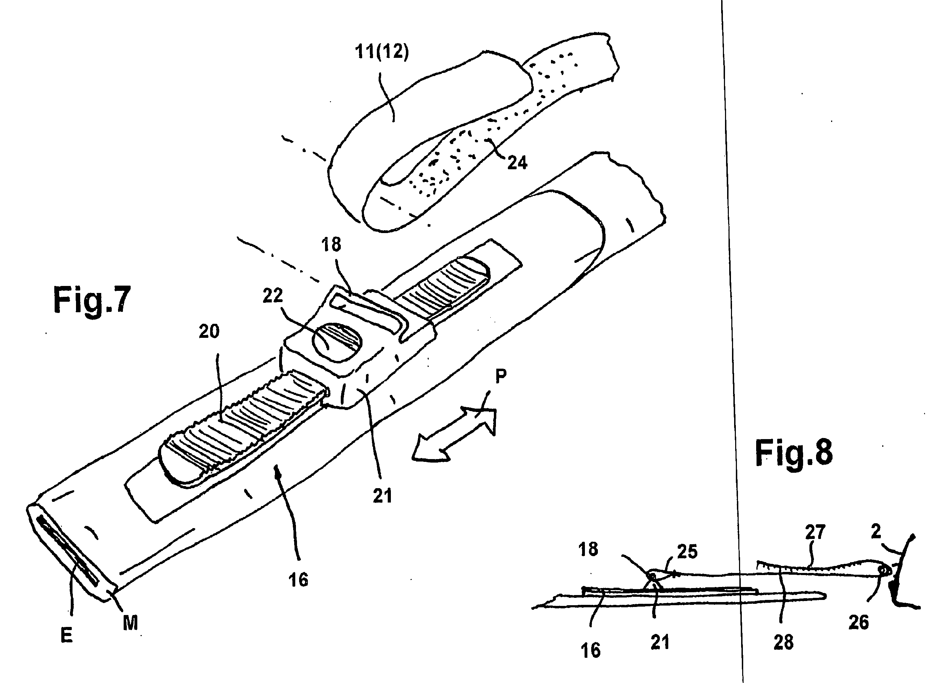

Figur 7 zeigt eine Kopfbandvariante mit einem, aus einem geschäumten Material gebildeten, Polsterkörper M und einer darin eingebetteten zugsteifen Lage E. Die zugsteife Lage E trägt eine Arretiereinrichtung 16, die eine mit Rastzähnen 20 versehene Führungsbahn bildet. Auf dieser Führungsbahn ist ein Schlittenelement 21 angeordnet. Das Schlittenelement 21 kann unter Betätigung eines Druckknopfs 22 und hierdurch verursachte Entriegelung des nicht näher dargestellten Arretiermechanismus in die durch das Pfeilsymbol P angedeuteten Richtungen verschoben werden. Das Schlittenelement 21 trägt einen Laschenabschnitt 18, durch welchen ein Bandabschnitt 11,12 der Kopfbandeinrichtung hindurchführbar ist. Der Bandabschnitt 11, 12 ist mit einer Klettverschlusseinrichtung 24 versehen, durch welche eine grobe Voreinstellung der effektiven Länge des jeweiligen Kopfbandabschnitts 3, 4 bewerkstelligbar ist.

Alternativ zu der hier gezeigten Anbindung des Kopfbandabschnitts 11, 12 an den Laschenabschnitt 18 ist es auch möglich, das Schlittenelement 21, wie in Figur 8 schematisch dargestellt, mit einer Atemmaske 2 zu koppeln und zwar indem der Laschenabschnitt 18 in eine fest vernähte Bandlasche 25 eingebettet ist und die Ankoppelung an die Atemmaske 2 über eine, durch einen Haltebügel 26 der Atemmaske 2 hindurchgeführte Bandlasche 27 bewerkstelligt ist, die wiederum durch eine Klettverschlusseinrichtung 28 lösbar ausgebildet ist. Das Schlittenelement 21 ist entlang der Arretiereinrichtung 16 verschiebbar. Die Arretiereinrichtung 16 kann auch durch eine im Bandkorpus versenkt ausgebildete Bahneinrichtung (Figur 6a) verwirklicht sein.

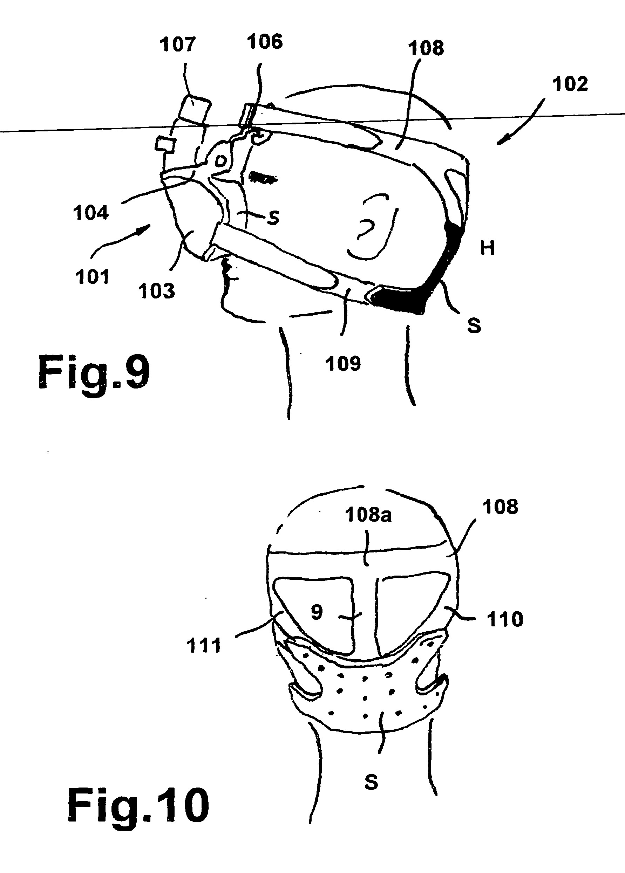

In Figur 9 ist ein Atemmaskenanwender gezeigt, an welchem eine weitere Atemmaskenanordnung 101 über eine, hier als Kopfbandanordnung ausgeführte, Applikationsvorrichtung 102 fixiert ist. Die Atemmaskenanordnung 101 umfasst einen Maskenbasiskörper 103, der über eine Dichtlippe 105 abdichtend auf dem Nasenbereich des Anwenders aufsitzt. Der Maskenbasiskörper 103 ist über eine Rahmenstruktur und eine mit dieser gekoppelte Stirnauflageeinrichtung 106 am Anwender fixiert. Die Zufuhr des Atemgases zu dem, durch den Maskenbasiskörper 103 definierten Maskeninnenraum, kann über die hier nur skizzenhaft dargestellte Anschlusseinrichtung 107 erfolgen.

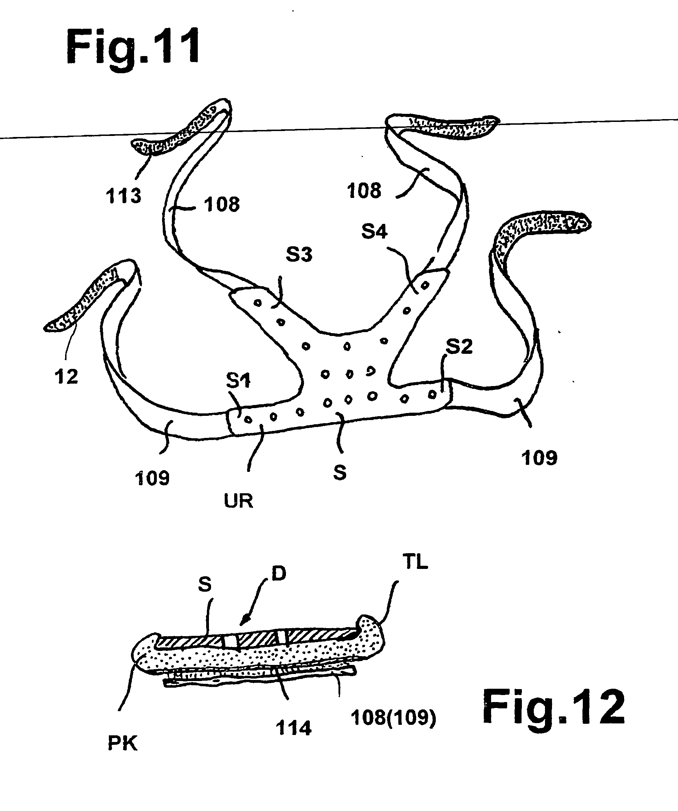

Die Applikationsvorrichtung 102 umfasst einen oberen Kopfbandabschnitt 108, der sich vom Hinterkopfbereich H ausgehend in den Bereich der Stimauflageeinrichtung 106 erstreckt. Weiterhin umfasst die Applikationsvorrichtung auch einen unteren Kopfbandabschnitt 109, der sich vom Nackenbereich des Anwenders ausgehend über dessen Wangenknochen in den Bereich des Maskenbasiskörpers 103 erstreckt. Die Koppelung des jeweiligen Kopfbandabschnitts 108, 109 mit der Atemmaskenanordnung 101 ist bei dieser Ausführungsform über Klettverschlussstrukturen bewerkstelligt, wie diese nachfolgend noch in Verbindung mit Figur 11 beschrieben werden. Die Applikationsvorrichtung ist mit einer Stützstruktur S versehen, die bei diesem Ausführungsbeispiel durch ein formsteifes Plattenmaterial gebildet ist. Das formsteife Plattenmaterial ist aus einem Werkstoff gefertigt, der unter bestimmten Bedingungen plastisch verformbar und damit an die individuelle Hinterkopftektur des Anwenders anpassbar ist. Bei diesem Ausführungsbeispiel wird die Anpassbarkeit erreicht, indem das zur Bildung der Stützstruktur vorgesehene Material ein thermo-verformbares Kunststoffmaterial ist. Die Materialeigenschaften dieses Kunststoffmaterials sind derart abgestimmt, dass dieses bei Temperaturen im Bereich ab ca. 50° Celsius plastisch verformbar ist. Durch die Verwendung der erfindungsgemäß an die individuelle Hinterkopftektur angepassten Stützstruktur wird es möglich, die zur Applikation der Atemmaskenanordnung 101 erforderlichen Haltekräfte in einer, unter physiologischen Gesichtspunkten, vorteilhaften Weise aufzubringen. Durch die Anpassung der Stützstruktur S an die individuelle Kopfform des Anwenders wird es insbesondere möglich, den Verlauf der unteren Kopfbandanordnung 109 vorteilhaft festzulegen und zudem eine hohe Rutsch- und Verdrehsicherheit zu erreichen.

Figur 10 zeigt eine Ansicht der vorangehend beschriebenen Applikationsvorrichtung von hinten. Wie aus dieser Ansicht ersichtlich, ist der obere Kopfbandabschnitt 8 über den Hinterkopfbereich des Anwenders geführt. Der den Hinterkopfbereich überbrückende Abschnitt 8a des oberen Kopfbandabschnitts ist durch einen senkrechten Steg 9 sowie durch Diagonalstege 110, 111 mit der im Hinterkopfbereich des Anwenders verlaufenden unteren Bandanordnung der Applikationsvorrichtung 102 gekoppelt. Die Kopfbandeinrichtung ist im Hinterkopfbereich des Patienten mit jener Stützstruktur S ausgestattet. Diese Stützstruktur S ist an die individuelle Hinterkopfform des Anwenders angepasst. Die Stützstruktur S ist mit Durchbrechungen versehen, um hierdurch eine erhöhte Dampfdurchlässigkeit zu erreichen.

Wie aus Figur 11 ersichtlich, umfasst die Stützstruktur S einen unteren Randabschnitt UR, der Schenkelabschnitte S1, S2 aufweist, die derart angeordnet sind, dass diese den ihnen jeweils zugeordneten Bandabschnitt 109 derart führen, dass sich dieser Bandabschnitt in vorteilhafter Weise über den Wangenbereich des Maskenanwenders erstreckt. Die Stützstruktur weist bei diesem Ausführungsbeispiel zwei weitere Schenkelabschnitte S3, S4 auf, die dazu dienen, die oberen Bandabschnitten 108 der Kopfbandeinrichtung zu führen.

Die Kopfbandabschnitte 108, 109 sind mit Klettverschlusseinrichtungen 112, 113 versehen, die dazu dienen, die Kopfbandabschnitte 8, 9 lösbar mit entsprechenden Laschenabschnitten der Atemmaskenanordnung 101 zu koppeln.

In Figur 12 ist in Form einer vereinfachten Querschnittsskizze eine bevorzugte Ausführungsform der Applikationsvorrichtung im Bereich der Stützstruktur S dargestellt. Die Stützstruktur S ist mit einem Polsterkörper PK versehen. Der Polsterkörper PK ist bei diesem Ausführungsbeispiel aus einem Schaumstoffmaterial gebildet, das in eine textile Lage TL eingebettet ist. Die textile Lage TL kann derart ausgebildet sein, dass diese eine Koppelungsfläche 114 bereitstellt, die es ermöglicht, die derart ausgebildete, gepolsterte Stützstruktureinrichtung lösbar über eine Klettverschlusseinbindung mit der Kopfbandeinrichtung 108, 109 zu koppeln. Die Stützstruktur S ist mit Durchbrechungen D versehen, zur Erhöhung der Dampfdurchlässigkeit der Stützstruktur S.

Die Erfindung ist nicht auf die vorangehend beschriebenen Ausführungsbeispiele beschränkt.

Alternativ zu der Bewerkstelligung der Stützstruktur S durch ein thermo-verformbares Material ist es auch möglich, die Stützstruktur aus einem Material zu fertigen, das in anderer Weise an die Hinterkopfform des Anwenders anpassbar ist und diese individuelle Form hinreichend formstabil beibehält. Dies kann insbesondere erreicht werden durch Verwendung aushärtender Werkstoffe, wie beispielsweise Harze. Die Stützstruktur S kann derart vorgeformt sein, dass diese einen Krümmungsverlauf aufweist, der im Grunde der wahrscheinlichsten Hinterkopfgestalt Rechnung trägt, so dass eine weitere Anpassung ggf. nicht erforderlich ist.

Das Konzept der Verwendung einer temporär plastifizierbaren formsteifen Struktur kann in vorteilhafter Weise in Kombination mit dem ebenfalls erfindungsgemäßen Konzept der Bildung des Bandkorpus durch Schaum-Spritzformen Anwendung finden. Insbesondere ist es möglich, die hinsichtlich des Schaum-Spritzformkonzeptes genannten zugsteifen Strukturen zumindest im Hinterkopfbereich aus einem temporär, z.B. unter Wärmeeinwirkung plastifizierbaren oder zumindest hinreichend anformbaren Material zu fertigen.

## Patentansprüche

1. Kopfbandeinrichtung zur Applikation einer Atemmaske an einem Anwender mit einem flexiblen Bandkorpus, der obere und untere Bandabschnitte (3,4) aufweist, zur Übertragung der zur Applikation der Atemmaske erforderlichen Maskenhaltekräfte, wobei der Bandkorpus zumindest abschnittsweise aus einem geschäumten Kunststoffmaterial gefertigt ist, **dadurch gekennzeichnet, dass** der Bandkorpus mit Schliessmitteln versehen ist, zur Ankoppelung des Bandlaschenabschnitts oder Maskenbefestigungselementes an den Bandkorpus in einstellbar veränderbarer Weise, und die Schliessmittel eine Schlitteneinrichtung umfassen, die an der Kopfbandeinrichtung in unterschiedliche Schliessstellungen bringbar ist.

2. Kopfbandeinrichtung nach Anspruch 1, **dadurch gekennzeichnet, dass** das Kunststoffmaterial im Bereich seiner Aussenfläche verhautet ist.

3. Kopfbandeinrichtung nach wenigstens einem der Ansprüche 1 bis 2, **dadurch gekennzeichnet, dass** der Bandkorpus mit einer zugsteifen Einlage versehen ist.

4. Kopfbandeinrichtung nach wenigstens einem der Ansprüche 1 bis 3, **dadurch gekennzeichnet, dass** der Bandkorpus mit einer formstabilen Einlage versehen ist.

5. Kopfbandeinrichtung nach wenigstens einem der Ansprüche 1 bis 4, **dadurch gekennzeichnet, dass** die Schliessmittel eine Arretierstruktur umfassen, die integral mit der Kopfbandeinrichtung ausgebildet ist.

6. Kopfbandeinrichtung nach wenigstens einem der Ansprüche 1 bis 5, **dadurch gekennzeichnet, dass** die Schlitteneinrichtung einen Arretiermechanismus umfasst, zur Arretierung der Schlitteneinrichtung an der Arretierstruktur.

7. Kopfbandeinrichtung nach wenigstens einem der Ansprüche 1 bis 6, **dadurch gekennzeichnet, dass** der obere Bandabschnitt (3) sich in den Stirnbereich erstreckt und der untere Bandabschnitt (4) sich hin zur Atemmaske erstreckt.

8. Kopfbandeinrichtung nach wenigstens einem der Ansprüche 1 bis 7, **dadurch gekennzeichnet, dass** der Bandlaschenabschnitt oder das Maskenbefestigungselement als längenverstellbare Bandabschitte (11,12) ausgeführt sind.

9. Kopfbandeinrichtung nach wenigstens einem der Ansprüche 1 bis 8, **dadurch gekennzeichnet, dass** der Bandlaschenabschnitt oder das Maskenbefestigungselement als zur Atemmaske vordringende Bandabschnitte (11,12) ausgeführt sind.

10. Verfahren zur Herstellung einer Kopfbandeinrichtung nach einem der Ansprüche 3 bis 9, bei welchem im Rahmen eines ersten Kunststoffspritzschritts ein zur Bildung einer zugsteifen Einlage vorgesehenes Kunststoffmaterial in einen Formwerkzeugraum eingespritzt wird und im Rahmen eines nachfolgenden Kunststoffspritzschritts ein porenbildendes Kunststoffmaterial in ein Formwerkzeug derart eingebracht wird, dass dieses die zugsteife Einlage unter Bildung eines Polsterabschnitts zumindest abschnittsweise ummantelt.

## Claims

1. A headband means for applying a breathing mask to a user and having a flexible band body comprising upper and lower band portions (3, 4) for transmitting the mask holding forces required for applying the breathing mask,
wherein at least portions of the band body are made of a foamed plastic material, **characterized in that**
the band body is provided with closure means for coupling a band bracket portion or mask fixing element to the band body in an adjustable manner, and
the closure means comprise a slide means which can be brought in different closing positions on the headband means.

2. The headband means according to claim 1, **characterized in that** the plastic material has a skin in the area of its outer surface.

3. The headband means according to at least one of claims 1 or 2, **characterized in that** the band body is provided with a tensile proof insert.

4. The headband means according to at least one of claims 1 to 3, **characterized in that** the band body is provided with a dimensionally stable insert.

5. The headband means according to at least one of claims 1 to 4, **characterized in that** the closure means comprise a locking structure which is formed integrally with the headband means.

6. The headband means according to at least one of claims 1 to 5, **characterized in that** the slide means comprises a locking mechanism for locking the slide means on the locking structure.

7. The headband means according to at least one of claims 1 to 6, **characterized in that** the upper band portion (3) extends to the forehead area and the lower band portion (4) extends towards the breathing mask.

8. The headband means according to at least one of claims 1 to 7, **characterized in that** the band bracket portion or mask fixing element are configured as length adjustable band portions (11, 12).

9. The headband means according to at least one of claims 1 to 8, **characterized in that** the band bracket portion or mask fixing element are configured as band portions (11, 12) extending towards the breathing mask.

10. A process for producing a headband means of any of the claims 3 to 9, in which a plastic material intended for forming a tensile proof insert is injected in a molding tool space within the scope of a first plastic injection step, and within the scope of a subsequent plastic injection step a pore-forming plastic material is introduced in a molding tool in such a manner that it covers the tensile proof insert at least partially thereby forming a cushion portion.

## Revendications

1. Dispositif de bandeau serre-tête pour l'application d'un masque respiratoire sur un utilisateur, avec un corps formant bandeau souple qui présente des segments de bandeau supérieur et inférieur (3, 4), pour la transmission des forces de maintien du masque nécessaires à l'application du masque respiratoire, dans lequel le corps formant bandeau est, du moins par endroits, fabriqué dans un matériau plastique expansé, ainsi **caractérisé en ce que** le corps formant bandeau est pourvu de moyens de fermeture pour rattacher le segment à languette ou l'élément de fixation du masque au corps formant bandeau d'une manière réglable et modifiable, et **en ce que** les moyens de fermeture comprennent un dispositif de déplacement qui peut être amené dans différentes positions de fermeture sur le dispositif de bandeau serre-tête.

2. Dispositif de bandeau serre-tête selon la revendication 1, **caractérisé en ce que** le matériau plastique est pourvu d'un revêtement au niveau de sa surface extérieure.

3. Dispositif de bandeau serre-tête selon au moins l'une quelconque des revendications 1 à 2, **caractérisé en ce que** le corps formant bandeau est pourvu d'une armature résistante à la traction.

4. Dispositif de bandeau serre-tête selon au moins l'une quelconque des revendications 1 à 3, **caractérisé en ce que** le corps formant bandeau est pourvu d'une armature indéformable.

5. Dispositif de bandeau serre-tête selon au moins l'une quelconque des revendications 1 à 4, **caractérisé en ce que** les moyens de fermeture comprennent une structure d'arrêt qui est formée d'un seul tenant avec le dispositif de bandeau serre-tête.

6. Dispositif de bandeau serre-tête selon au moins l'une quelconque des revendications 1 à 5, **caractérisé en ce que** le dispositif de déplacement comprend un mécanisme d'arrêt pour son blocage contre la structure d'arrêt.

7. Dispositif de bandeau serre-tête selon au moins l'une quelconque des revendications 1 à 6, **caractérisé en ce que** le segment de bandeau supérieur (3) s'étend dans la zone frontale et **en ce que** le segment de bandeau inférieur (4) s'étend jusqu'au masque respiratoire.

8. Dispositif de bandeau serre-tête selon au moins l'une quelconque des revendications 1 à 7, **caractérisé en ce que** le segment à languette ou l'élément de fixation du masque sont réalisés sous la forme de segments de bandeau réglables dans la longueur (11, 12).

9. Dispositif de bandeau serre-tête selon au moins l'une quelconque des revendications 1 à 8, **caractérisé en ce que** le segment à languette ou l'élément de fixation du masque sont réalisés sous la forme de segments de bandeau (11, 12) s'avançant vers le masque respiratoire.

10. Procédé de fabrication d'un dispositif de bandeau serre-tête selon l'une quelconque des revendications 3 à 9, dans lequel, dans le cadre d'une première étape d'injection de plastique, on injecte un matériau plastique, prévu pour la formation d'une armature résistante à la traction, dans un espace de moule de formage et, dans le cadre d'une étape d'injection de plastique suivante, on place un matériau plastique à formation de pores dans un moule de formage de telle sorte que ce dernier enveloppe, du moins par endroits, l'armature résistante à la traction en formant une section rembourrée.
